# EUROPEAN PATENT APPLICATION

(11) **EP 1 437 406 A2**
(43) Date of publication of application: **14.07.2004**
(21) Application number: 02775215.3
(22) Date of filing: 20.09.2002
(51) Int. Cl.: C12N 15/09, C12N 5/00, C12Q 1/02

(54) **PLURIPOTENT STEM CELLS ORIGINATING IN SKELETAL MUSCLE INTESTINAL TISSUE**

(30) Priority: 20.09.2001 JP 2001286332; 09.05.2002 JP 2002133575
(71) Applicant: KYOWA HAKKO KOGYO CO., LTD., Chiyoda-ku, Tokyo 100-8185 (JP); Tamaki, Tetsuro, Isehara-shi, Kanagawa 259-1145 (JP); Ando, Kiyoshi, Chigasaki-shi, Kanagawa 253-0037 (JP)
(72) Inventor: TAMAKI, Tetsuro, Isehara-shi, Kanagawa 259-1145 (JP); ANDO, Kiyoshi, Chigasaki-shi, Kanagawa 253-0037 (JP); AKATSUKA, Akira, Isehara-shi, Kanagawa 259-1131 (JP); NAKAMURA, Yoshihiko, Hiratsuka-shi, Kanagawa 259-1212 (JP); HOTTA, Tomomitsu, Nagoya-shi, Aichi 468-0015 (JP); SAKURADA, K., c/o Kyowa Hakko Kogyo Co., Ltd., Chiyoda-ku, Tokyo 100-8185 (JP)
(74) Representative: VOSSIUS & PARTNER
(86) International application number: PCT/JP2002/009702
(87) International publication number: WO 2003/027281

(57) **Abstract**

The present invention relates to multipotent stem cells derived from the interstitial tissues of skeletal muscle. The multipotent stem cell of the present invention is capable of differentiating into skeletal muscle cells, smooth muscle cells, cardiomyocytes, blood cells, vascular endothelial cells, adipocytes, osteoblasts, nervous cells, hepatocytes and pancreatic cells, and is useful for regeneration of tissues and cells and treatment for cardiac failure, hepatic insufficiency, renal insufficiency, leukemia, nerve degeneration disease, arthritis, diabetes, arteriosclerosis, and the like.

SEQUENCE LISTING

<110> KYOWA HAKKO KOGYO CO., LTD.
<110> TETSURO TAMAKI
<110> KIYOSHI ANDO

<120>

<130> 11417W01

<140>
<141>

<150> JP2001-286332
<151> 2001-09-20

<150> JP2002-133575
<151> 2001-05-09

<160> 19

<170> PatentIn Ver. 2.0

<210> 1
<211> 22
<212> DNA
<213> Artificial Sequence
<220>
<223> Synthetic DNA

<400> 1
acatagactt gacaggcccc ga 22

<210> 2
<211> 22
<212> DNA
<213> Artificial Sequence
<220>
<223> Synthetic DNA

<400> 2
agaccttcga tgtagcggat gg 22

<210> 3
<211> 22
<212> DNA
<213> Artificial Sequence
<220>
<223> Synthetic DNA

<400> 3
ggtcaaccaa gctttcgaga cg                                                22

<210> 4
<211> 21
<212> DNA
<213> Artificial Sequence
<220>
<223> Synthetic DNA

<400> 4
cggagctttt atctgcagca c                                                 21

<210> 5
<211> 19
<212> DNA
<213> Artificial Sequence
<220>
<223> Synthetic DNA

<400> 5
attctgcgga gtgccatca                                                    19

<210> 6
<211> 20
<212> DNA
<213> Artificial Sequence
<220>
<223> Synthetic DNA

<400> 6
tgttccaaat gctggctgag                                                   20

<210> 7
<211> 21

<212> DNA
<213> Artificial Sequence
<220>
<223> Synthetic DNA

<400> 7
tacgtccatc gtggacagca t                                                21

<210> 8
<211> 21
<212> DNA
<213> Artificial Sequence
<220>
<223> Synthetic DNA

<400> 8
tcagctaaat tccctcgctg g                                                21

<210> 9
<211> 21
<212> DNA
<213> Artificial Sequence
<220>
<223> Synthetic DNA

<400> 9
tggagcgtca gccagattaa c                                                21

<210> 10
<211> 21
<212> DNA
<213> Artificial Sequence
<220>
<223> Synthetic DNA

<400> 10
ttgtcccgaa ggtcctcttg t                                                21

<210> 11
<211> 21
<212> DNA
<213> Artificial Sequence
<220>

<223> Synthetic DNA

<400> 11
ccaagccgcg tatgtcagta a                                                21

<210> 12
<211> 19
<212> DNA
<213> Artificial Sequence
<220>
<223> Synthetic DNA

<400> 12
aataagtcga cgcgctgca                                                   19
<210> 13
<211> 21
<212> DNA
<213> Artificial Sequence
<220>
<223> Synthetic DNA

<400> 13
gaaagccaaa cacagcatcg a                                                21

<210> 14
<211> 13
<212> DNA
<213> Artificial Sequence
<220>
<223> Synthetic DNA

<400> 14
accctgatgc atggttgatg g                                                21

<210> 15
<211> 24
<212> DNA
<213> Artificial Sequence
<220>
<223> Synthetic DNA

<400> 15
cctggaaccc acgaccacgg tgtc                                             24

<210> 16
<211> 20
<212> DNA
<213> Artificial Sequence
<220>
<223> Synthetic DNA

<400> 16
aacgtccaag gcttactttg                                                    20

<210> 17
<211> 21
<212> DNA
<213> Artificial Sequence
<220>
<223> Synthetic DNA

<400> 17
aacaccccag ccatgtacgt a                                                  21

<210> 18
<211> 21
<212> DNA
<213> Artificial Sequence
<220>
<223> Synthetic DNA

<400> 18
aaggaaggct ggaaaagagc c                                                  21

<210> 19
<211> 1132
<212> PRT
<213> Homo sapiens

<400> 19

## Description

### Technical Field

The present invention relates to a stem cell derived from the interstitial tissues of skeletal muscle, which is capable of differentiating into skeletal muscle cells, smooth muscle cells, cardiomyocytes, blood cells, vascular endothelial cells, adipocytes, osteoblasts, nervous cells, hepatocytes, pancreatic cells and the like. Also, the present invention relates to a medicament such as an agent for regenerating tissues or cells, which comprises the stem cell and to a method for using the cell.

### Background Art

Satellite cells in muscles are known as specific cells having the muscle differentiation inducing ability acting upon the growth, repair and maintenance of muscles after birth [*Dev. Biol.,* 218; 115-124 (2000)]. Satellite cells in mice occupy about 30% of the nuclei existing inside of the lamina membrane in muscle fibers at the time of birth, but are reduced to about 5% two months thereafter [*Myogenesis* (AG Engel and C. Franszini-Amstrong, ads, New York: McGraw-Hill), pp. 97-118 (1994)]. The reduction of satellite cells reflects growth of muscles after birth [*Muscle Nerve,* 6*,* 574-580 (1983)]. In addition, the satellite cells have a property that they are present in mature adult muscles and in contact with muscle fibers at a position contiguous to the inside of the lamina membrane [*Myogenesis* (AG Engel and C. Franszini-Amstrong, ads, New York: McGraw-Hill), pp. 97-118 (1994)].

In mice, satellite cells terminate cell division and enter into resting stage after an elapse of two months. However, it is known that the satellite cells are activated by various stimuli such as stretch, motion, injury and electrical stimulus [*Muscle Nerve, 8,* 217-222 (1985); *Int. J. Sports Med.,* 9, 297-299 (1988); *Muscle Nerve,* 17, 608-613 (1994); *Myogenesis* (AG Engel and C. Franszini-Amstrong, ads, New York: McGraw-Hill), pp. 97-118 (1994)]. Muscle precursor cells as progenies of satellite cells generate two or more of cell division and then differentiate into muscle cells to cause fusion with adjacent muscle fibers. On the other hand, since the number of the satellite cells themselves in the resting stage does not change even when degeneration and regeneration are repeated, the satellite cells are maintained in a constant numbers by self-replication [*Muscle Nerve,* 6, 574-580 (1983); *Mech Aging Dev.,* 30, 63-72 (1985): *Anat. Embryol.,* 180, 471-478 (1989)]. Accordingly, satellite cells are stem cells having unipotency and have properties which are biologically and biochemically different from those of muscle precursor cells as progenies thereof [*Mol. Cell. Biol. Hum. Dis.,* 3, 210-256 (1993); *Myogenesis* (AG Engel and C. Franszini-Amstrong, ads, New York: McGraw-Hill), pp. 97-118 (1994)].

Based on the above, it is considered that satellite cells are present in adult muscles and have a physiological function for inducing muscle precursor cells which become muscle in the future.

In addition to the satellite cells, the presence of a stem cell population called side population cells (SP cells) having pluripotency in the skeletal muscle has recently been found. The SP cells can be separated according to FACS (fluorescence-activated cell sorting) by using a property that it discharges a Hoechst dye (Hoechst 33342) [*Nature,* 401, 390-394 (1999), *Proc. Natl. Acad Sci. USA,* 96,14482-14486 (1999)]. It has been shown that SP cells separated from a tissue are differentiated into all of the principal blood cells when they are transplanted in a mouse whose bone marrow was destructed by radioactive rays [*Proc. Natl. Acad. Sci. USA,* 96, 14482-14486 (1999)]. Furthermore, SP cells separated from the marrow and muscles have a property that they also have the ability to regenerate muscles. However, only the SP cell derived from muscles can become a satellite cell [*Nature,* 401, 390-394 (1999)]. It has been shown also that SP cells differentiate into a desmin-positive muscle when put under an appropriate culture condition [*Nature,* 401, 390-394 (1999)]. On the other hand, it has been found that satellite cells in muscles are completely disappeared and SP cells are increased in a Pax7 gene knockout mouse [*Cell,* 102, 777-786 (2000)].

This result suggests that a multipotent stem cell different from satellite cells is present in the skeletal muscle. However, the specific location of the stem cell different from satellite cells in the skeletal muscle and the kinds of its physiological function and differentiation potency have not been found.

Also, it is known that satellite cells are cells which are m-cadherin-positive and CD34-negative, and it is known also that the SP cells separated from skeletal muscle are cells which are CD34-negative, Sca-1-positive, c-kit-negative and CD45-negative [*Nature,* 401, 390-394 (1999)].

In entering an aging society, tissue disorders and organ insufficiencies caused by aging, chronic diseases, injuries and the like are becoming serious problems. At the present, there are no methods for treating disorders of tissues and organs with drugs, so that patients become a bedridden or care-requiring state accompanied by the advance of diseases. On the other hand, organ transplantation also has a problem of infections and rejection reactions in addition to the insufficient donors. Based on the advance in adult stem cell biology in recent years, it has been suggested that somatic stem cells are present in various tissues of adult bodies. In addition, it has been considered that reduction of these somatic stem cells accompanied by aging is the basic cause of the tissue disorders and organ insufficiencies of the aged. Thus, it is considered that a treatment for supplementing lost somatic stem cells is effective in treating tissue disorders and organ insufficiencies [*Saibo (Cell),* 33, 101-104 (2001)].

### Disclosure of the Invention

The present invention relates to the following items (1) to (24).
(1) A multipotent stem cell which is derived from the interstitial tissues of skeletal muscle.
(2) The multipotent stem cell according to (1), which is c-met-positive.
(3) The multipotent stem cell according to (1) or (2), which is CD34-positive.
(4) The multipotent stem cell according to any one of (1) to (3), which is Scal-positive.
(5) The multipotent stem cell according to any one of (1) to (4), which is m-cadherin-negative.
(6) The multipotent stem cell according to any one of (1) to (5), which is CD45-negative.
(7) The multipotent stem cell according to any one of (1) to (6), which is CD14-negative, CD31-negative, CD49d-negative, CD117-negative, FLK-1-negative and CD144-negative.
(8) The multipotent stem cell according to (1), which is CD34-negative, CD45-negative, m-cadherin-negative and MyoD-negative.
(9) The cell according to any one of (1) to (8), which is a multipotent stem cell capable of differentiating into at least skeletal muscle cells.
(10) The cell according to any one of (1) to (9), which is a multipotent stem cell capable of differentiating into at least a skeletal muscle cell, a smooth muscle cell, a cardiomyocyte, a vascular endothelial cell and an adipocyte.
(11) The cell according to any one of (1) to (10), which is a multipotent stem cell capable of differentiating into at least a skeletal muscle cell, a smooth muscle cell, a cardiomyocyte, a blood cell, a vascular endothelial cell, an adipocyte, a cartilage cell, an osteoblast, a nervous cell, a hepatocyte, a pancreatic cell, a nephrocyte, a prostatic cell, a mammary gland cell, a small intestine epidermal cell and a corneal cell.
(12) The cell according to any one of (1) to (11), wherein the skeletal muscle is derived from a mammal.
(13) The cell according to (12), wherein the mammal is selected from human and rat.
(14) A medicament which comprises the cell according to any one of (1) to (13).
(15) The medicament according to (14), wherein the medicament is an agent for regenerating a tissue or a cell.
(16) The medicament according to (14), wherein the medicament is an agent for treating organ insufficiencies.
(17) The medicament according to (15), wherein the tissue or cell is a tissue or cell of muscle, heart, blood, blood vessel, bone, joint, pancreas, liver and nerve system.
(18) An antibody which specifically recognizes the cell according to any one of (1) to (13).
(19) A method for purifying the cell according to any one of (1) to (13) from human skeletal muscle, which comprises using the antibody according to (18).
(20) A method for screening a substance which proliferates the cell according to any one of (1) to (13), which comprises using the cell.
(21) A method for screening a substance which induces differentiation of the cell according to any one of (1) to (13) into various cells by using the cell according to any one of (1) to (13).
(22) The screening method according to (21), wherein the various cells are selected from a skeletal muscle cell, a smooth muscle cell, a cardiomyocyte, a blood cell, a vascular endothelial cell, an adipocyte, an osteoblast, a cartilage cell, a nervous cell, a hepatocyte, a pancreatic cell, a nephrocyte, a prostatic cell, a mammary gland cell, a small intestine epidermal cell, a skin cell and a corneal cell.
(23) A method for immortalizing the cell according to any one of (1) to (13).
(24) A method for separating the cell according to any one of (1) to (13), wherein a sphere is formed.

The stem cell is a cell which have self-replication ability and is also capable of differentiating into two or more cells, and is also called multipotent stem cell.

Among the multipotent stem cells, a cell which is capable of differentiating into all cells in the living body is called pluripotent stem cell, and a cell which is further capable of reconstructing an individual is called totipotent stem cell.

The stem cells include ES cells (embryonic stem cells) obtained from blastocyst, somatic stem cells present in tissues, reproductive stem cells which differentiate into reproductive cells, and the like. The somatic stem cells and reproductive stem cells are further classified into two kinds, adult-derived and fetus-derived. In spite of the presence of varied spans of life peculiar to respective cells constituting the human body, significant changes in the number of cells in tissues and organs do not occur throughout the life. This is because regeneration and new formation of cells are carried out throughout the life under a strict control in order to compensate for the perished cells. The cells having the activity to carry out regeneration and new formation of cells in the adult tissues in this way are somatic stem cells (tissue stem cells).

The multipotent stem cell of the present invention is classified into a novel adult somatic stem cell and has the self-replication ability and the ability to differentiate into somatic cells. The somatic cells may be any cells which construct adult tissues, and examples include those cells which generally do not have the self-replication ability, such as skeletal muscle cells, smooth muscle cells, cardiomyocytes, blood cells, vascular endothelial cells, adipocytes, osteoblasts, cartilage cells, nervous cells, hepatocytes, pancreatic cells, nephrocytes, prostatic cells, mammary gland cells, small intestine epidermal cells, skin cells and corneal cells.

The multipotent stem cell of the present invention includes cells having any one or plural properties of the following (1) to (6) regarding the cell surface antigen and cells having the property of (7).
(1) c-met-positive;
(2) CD34-positive;
(3) Scal-positive;
(4) m-cadherin-negative;
(5) CD45-negative;
(6) CD14-negative, CD31-negative, CD49d-negative, CD117-negative, FLK-1-negative and CD144-negative;
(7) CD34-negative, CD45-negative, m-cadherin-negative and MyoD-negative.

The multipotent stem cell of the present invention is derived from the interstitial tissues of skeletal muscle and is capable of differentiating into at least skeletal muscle cells. That is, the multipotent stem cell of the present invention is capable of differentiating into skeletal muscle cells and other cells, preferably at least skeletal muscle cells, smooth muscle cells, cardiomyocytes, vascular endothelial cells and adipocytes, more preferably at least skeletal muscle cells, smooth muscle cells, cardiomyocytes, blood cells, vascular endothelial cells, adipocytes, cartilage cells, osteoblasts, nervous cells, hepatocytes, pancreatic cells, nephrocytes, prostatic cells, mammary gland cells, small intestine epidermal cells and corneal cells.

The multipotent stem cell of the present invention is derived from the interstitial tissues of skeletal muscle, and as the skeletal muscle, skeletal muscles of mammals such as mouse, rat, guinea pig, hamster, rabbit, cat, dog, sheep, pig, cattle, goat, monkey and human are used. When the multipotent stem cell of the present invention is used for the treatment of human, it is preferably derived from a human.

The method for separating and purifying the multipotent stem cell of the present invention is described below.

### 1 Method for separating interstitial cells of skeletal muscle from the living body

Although the method for obtaining multipotent stem cells from human skeletal muscle is not particularly limited, they can be obtained by the following method.

Connective tissues including muscles such as the lateral head of brachial biceps muscle and leg sartorius muscle of a patient requiring a cell therapy are excised by cutting the skin and then sutured. The total muscle thus obtained is made into a mince with scissors or a surgical knife, then suspended in a high concentration glucose medium containing 0.06% collagenase and 10% FBS and incubated at 37°C for 2 hours. After the cells are separated from the minced muscle, they are recovered by centrifugation and suspended in a high concentration glucose medium containing 10% FBS. A suspension of human skeletal muscle interstitial cells can be obtained by firstly passing the suspension through a microfilter of 40 µm pore size and then through a microfilter of 20 µm pore size.

Although the method for obtaining multipotent stem cells from a rat or mouse is not particularly limited, they can be obtained by the following method.

A rat or a mouse is sacrificed by cervical vertebra dislocation and thoroughly disinfected with 70% ethanol, and then femoral quadriceps muscle is obtained by cutting off the skin. The femoral quadriceps muscle is made into a mince with scissors or a surgical knife and then suspended in a high concentration glucose medium containing 0.06% collagenase and 10% FBS and incubated at 37°C for 2 hours. After cells separated from the minced muscle are recovered, they are recovered by centrifugation and suspended in a high concentration glucose medium containing 10% FBS. A suspension of rat or mouse skeletal muscle interstitial cells can be obtained by firstly passing the suspension through a microfilter of 40 µm pore size and then through a microfilter of 20 µm pore size.

### 2. Method for obtaining multipotent stem cells

The method for obtaining a cell expressing the surface antigen of interest from the suspension of skeletal muscle interstitial cells obtained in the above item 1 includes an FACS method using a flow cytometer having a sorting function [*Int. Immunol.,* 10(3), 275-283 (1998)], a method using magnetic beads and a panning method using an antibody capable of specifically recognizing the multipotent stem cell of the present invention [*J. Immunol.,* 141(8), 2797-2800 (1988)]. In addition, as the method for obtaining the multipotent stem cell of the present invention from a large amount of a culture medium or the like, the multipotent stem cell of the present invention can also be obtained by using a column packed with antibodies capable of specifically recognizing a molecule expressing on the cell surface (hereinafter referred to as "surface antigen") alone or in combination thereof.

The flow cytometer sorting method includes a water drop charging method, a cell capture method, and the like [*Unrestricted Flow Cytometer,* pp. 14-23, published by Shujunsha (1999)]. In both methods, an expressed amount of a cellular antigen can be determined by labeling an antibody capable of specifically recognizing a cell surface antigen with a fluorescence, measuring fluorescence of the combination of the labeled antibody with the antigen and then converting the fluorescence intensity into an electrical signal. Furthermore, it is possible to separate a cell expressing two or more surface antigens by a combination of the kinds of fluorescence to be used. The fluorescence includes FITC (fluorescein isothiocyanate), PE (phycoerythrin), APC (allo-phycocyanin), TR (TexasRed), Cy 3, CyChrome, Red 613, Red 670, PerCP, TRIColor, QuantumRed and the like [*Unrestricted Flow Cytometer,* pp. 3-13, published by Shujunsha (1999)].

The applicable FACS method using a flow cytometer includes a method in which a skeletal muscle interstitial solution is collected in accordance with the method described in the above item 1 from a skeletal muscle tissue excised from the living body and then the cells are separated by a method such as centrifugation and directly stained with an antibody and a method in which the cells are once cultured and grown in an appropriate medium and then stained with an antibody. In carrying out staining of cells, a sample of the cells of interest is firstly mixed with a primary antibody which recognizes the surface antigen and then incubated on ice for 30 minutes to 1 hour. When the primary antibody is labeled with a fluorescence, the cells are separated by the flow cytometer after washing. When the primary antibody is not labeled with a fluorescence, the cells reacted with the primary antibody are mixed with a fluorescence-labeled secondary antibody having affinity for the primary antibody after washing and again incubated on ice for 30 minutes to 1 hour, and after washing, cells stained with the primary antibody and secondary antibody are separated using the flow cytometer.

When a method using magnetic beads is employed, cells expressing the surface antigen of interest can be separated in a large quantity. Although its separation purity is not equal to that of the above flow cytometer-aided method, sufficiently high cell purity can be obtained by repeating the purification.

Specifically, a primary antibody is allowed to react with a skeletal muscle interstitial solution and, after removing unreacted primary antibody, the reacted primary antibody is bound to a secondary antibody bound to magnetic beads capable of specifically binding to the primary antibody. The remaining secondary antibody is removed by washing, and the cells are separated by a stand equipped with a magnet. Materials and equipment necessary for these operations are available from DYNAL.

The method using magnetic beads can also be used in removing unnecessary cells from a sample of cells. For more efficiently removing unnecessary cells, a method using StemSep available from Stem Cell Technologies (Vancouver, Canada) is used.

As the surface antigens, mentioned are a surface antigen of a hematopoietic cell, a surface antigen of a vascular endothelial cell, a surface antigen of a mesenchymal cell, a surface antigen of integrin, a matrix receptor, an adhesion molecule, a cytokine receptor and the like.

The surface antigen of hematopoietic cells includes CD34, c-kit (CD117), CD14, CD45, CD90, Sca-1, Ly6c, Ly6g, and the like. The surface antigen of vascular endothelial cells includes Flk-1, CD31, CD105, CD144, and the like. The surface antigen of mesenchymal cells includes CD140, and the like. The surface antigen of integrin includes CD49b, CD49d, CD29, CD41, and the like. The matrix receptor includes CD54, CD102, CD106, CD44, and the like. The adhesion molecule includes m-cadherin, and the like. The cytokine receptor includes c-met, and the like.

A cell of the interest can be obtained by using antibodies capable of recognizing the above mentioned surface antigens alone or in combination thereof.

For example, in order to obtain a cell which is CD34-positive, the skeletal muscle interstitial solution prepared in the above item 1 is applied to an anti-CD34 antibody-linked column to bind the CD34-positive cell to the anti-CD34 antibody, and thereafter, the cell of interest can be separated by eluting the CD34-positive cell from the anti-CD34 antibody.

The method for separating the multipotent stem cell of the present invention includes a method in which skeletal muscle interstitial cells are cultured in accordance with the following method and then a cell forming a sphere is separated. The sphere is a cell mass in a culture supernatant, and can be easily discriminated under a microscope from adhesive cells and suspending single cells.

As the medium to be used in the culturing of cells, a cell culture medium having a conventionally known composition [*Basic Tissue Culture Techniques,* 3rd edition, Asakura Shoten (1996)] can be generally used, but cell culture medium such as α-MEM, DMEM or IMDM supplemented with serum such as bovine serum in an amount of 5 to 20% is preferably used.

The culturing conditions may be any conditions, so long as the cells can be cultured. The cells are preferably cultured at a culture temperature of 33 to 37°C, and more preferably in an incubator filled with 5 to 10% carbon dioxide gas.

Culturing is carried out by adhering or suspending the cells to or in a usual tissue culture plastic culture dish. In the case where the cells are cultured by adhering them, the medium is removed when the cells are grown all over the culture dish, and the resulting cells are suspended by adding a trypsin-EDTA solution. The suspended cells can be further subjected to subculturing by washing with PBS or the cell culture medium, diluting 5-fold to 20-fold with the cell culture medium, and then pouring into a fresh culture dish.

Purity of the multipotent stem cell of the present invention obtained by the method described in the above can be examined by an FACS method which uses antibodies for the above mentioned surface antigens.

### 3. Examination method of the multipotent stem cell of the present invention

The multipotent stem cell of the present invention is examined by the following method.

The multipotent stem cell of the present invention separated by the method described in the above item 2 is diluted to give a degree of 1 cell/well in a 96 well culture plate, and the cell suspension is dispensed into each well. The cells are cultured, and the differentiation-induced cells are analyzed by the following method, to thereby examine the multipotent stem cell of the present invention.

The medium used in the culturing of cells includes a cell culture medium having a conventionally known composition [*Basic Tissue Culture Techniques,* 3rd edition, Asakura Shoten (1996)]. Preferably, a cell culture medium such as α-MEM, DMEM or IMDM supplemented with serum such as bovine serum in an amount of 5 to 20% is used.

The culture conditions may be any conditions, so long as the cells can be cultured. The cells are preferably cultured at 33 to 37°C, and are more preferably cultured in an incubator filled with 5 to 10% carbon dioxide gas.

Culturing is preferably carried out culturing by adhering or suspending the cells to or in a usual tissue culture plastic culture dish. In the case where the cells are cultured by adhering them, the medium is removed when the cells are grown all over the culture dish, and the resulting cells are suspended by adding a trypsin-EDTA solution. The suspended cells can be further subjected to subculturing by washing with PBS or the cell culture medium, diluting 5-fold to 20-fold with the cell culture medium, and pouring into a fresh culture dish.

When cells proliferate as a result of the culturing of cells by the above method, the cells are considered to have self-replication ability.

Also, whether the cells have multipotency can be examined by identifying differentiated cells randomly appeared from the cultured cells, or by making the culturing confluent so that the cells stop growth and start differentiation, and then identifying the differentiated cells.

The method for identifying differentiated cells includes a conventionally known method using an antibody which recognizes a marker of the differentiated cells or using a probe such as DNA/RNA *(Immunostaining-In situ Hybridization,* Supplement of Experimental Medicine, edited by Sumiharu. Noji, published by Yodosha).

The markers for identifying differentiated cells are shown below.

The marker for identifying neuron includes microtuble associate protein 2ab, neurofilament, and the like. The marker for identifying glia includes glial fibrillary acidic protein. The marker for identifying cardiac muscle includes Mkx2.5, GATA4, cardiac Troponin I, and the like. The marker for identifying muscle includes MyoD, and the like. The marker for identifying adipocyte includes peroxisome proliferation-activated receptor γ2, lipoprotein lipase, fatty acid-binding protein, and the like.. The marker for identifying vascular endothelium includes kdr/flkl, Low density lipoprotein receptor, and the like. The marker for identifying blood cell includes CD45. The marker for identifying bone includes alkaline phosphatase. The marker for identifying cartilage includes type II collagen. The marker for identifying pancreatic β cell includes insulin. The marker for identifying hepatocyte includes albumin.

Furthermore, another method for examining the multipotent stem cell of the present invention includes a method in which the multipotent stem cell of the present invention labeled with a marker such as GFP is transplanted into an NOD-SCID (nonobese diabetic/severe combined immunodeficient) mouse, and the differentiation induced cells are analyzed by the above immunostaining or *in situ* hybridization.

### 4. Method for culturing the multipotent stem cell of the present invention

The medium used in the culturing of the multipotent stem cells separated by the method described in the above item 2 includes a cell culture medium having a conventionally known composition [*Basic Tissue Culture Techniques,* 3rd edition, Asakura Shoten (1996)] can be generally used. Preferably, a cell culture medium such as α-MEM, DMEM or IMDM supplemented with serum such as bovine serum in an amount of 5 to 20% is used. The culture conditions may be any conditions, so long as the cells can be cultured. The cells are preferably cultured at a culture temperature of 33 to 37°C, and more preferably in an incubator filled with 5 to 10% carbon dioxide gas. Proliferation of the multipotent stem cell of the present invention is preferably carried out by adhering or suspending them to or in a usual tissue culture plastic culture dish. In the case where the cells are cultured by adhering them, the medium is removed when the cells are grown all over the culture dish, and the resulting cells are suspended by adding a trypsin-EDTA solution. The suspended cells can be subjected to subculturing by washing with PBS or the cell culture medium, diluting 5-fold to 20-fold with the cell culture medium, and pouring into a fresh culture dish.

### 5. Therapeutic agent for regenerating tissue or cell comprising the multipotent stem cell of the present invention

The multipotent stem cell of the present invention can be used for the regeneration of various tissues or cells or as an agent for treating various organ insufficiencies.

The organ insufficiencies are not particularly limited, so long as they are diseases which accompany destruction or degeneration of adult tissues and cells, and examples include cardiac failure, hepatic insufficiency, renal insufficiency, leukemia, nerve degeneration disease, arthritis, diabetes mellitus, arteriosclerosis and the like.

Various organ insufficiencies can be treated by differentiating and proliferating the multipotent stem cell of the present invention depending on the diseased position or size of the lesion of organs caused by the above diseases through *in vitro* culturing, and then transplanting the cell into the diseased position of respective organ. Alternatively, the multipotent stem cell of the present invention may be directly transplanted into the diseased position of respective organ.

Among the multipotent stem cells of the present invention, as the agent for treating cardiac failure, those which contain cells capable of differentiating into cardiomyocytes at high purity are mentioned. The cardiomyocytes include endocardial endothelial cells, cushion cells, ventricle type cardiomyocytes, atrium type cardiomyocytes, sinoatrial node cells and the like.

The multipotent stem cell of the present invention can be used for the treatment of cardiac failure by obtaining the cardiomyocyte of interest depending on the diseased position or size of the lesion of the heart through *in vitro* differentiation and proliferation, and then transplanting the cell into the diseased position of the heart. Alternatively, the multipotent stem cell of the present invention may be directly transplanted into the diseased position of the heart.

Among the multipotent stem cells of the present invention, as the agent for treating renal insufficiency, those which contain cells capable of differentiating into nephrons at high purity are mentioned. The nephrons include mesangial cells, podocytes, proximal tubule cells, distal tubule cells and the like.

The multipotent stem cell of the present invention can be used for the treatment of renal insufficiency by obtaining the kidney cell of interest depending on the diseased position or size of the lesion of the kidney through *in vitro* differentiation and proliferation, and transplanting the cell into the diseased position of the kidney. Alternatively, the multipotent stem cell of the present invention may be directly transplanted into the diseased position of the kidney.

Among the multipotent stem cells of the present invention, as the agent for treating nerve degeneration diseases, those which contain cells capable of differentiating into nervous cells at high purity are mentioned. Preferably, the nervous cells include central nerve system and peripheral nervous cells such as dopamine neuron and cholinergic neuron, and the like.

The multipotent stem cell of the present invention can be used for the treatment of nerve degeneration diseases by obtaining the nervous cell of interest depending on the diseased position or size of the lesion of a nerve through *in vitro* differentiation and proliferation, and transplanting the cell into the diseased position of the nerve. Alternatively, the multipotent stem cell of the present invention may be directly transplanted into the diseased position of the nerve.

Among the multipotent stem cells of the present invention, as the agent for treating arthritis, those which contain cells capable of differentiating into joint-forming cells at high purity are mentioned. Preferably, the joint-forming cells include cartilage cells, bone cells, ligament cells and the like.

The multipotent stem cell of the present invention can be used for the treatment of arthritis by obtaining a cell capable of differentiating into the joint forming cell of interest depending on the diseased position or size of the lesion of arthritis, through *in vitro* differentiation and proliferation, and transplanting the cell into the diseased position of the joint.

Among the multipotent stem cells of the present invention, as the agent for treating diabetes, those which contain cells capable of differentiating into pancreatic endocrine cells at high purity are mentioned. The pancreatic endocrine cells include β cells, α cells, δ cells and the like.

The multipotent stem cell of the present invention can be used for the treatment of diabetes mellitus by obtaining a cell capable of differentiating into the pancreatic endocrine cell of interest depending on the seriousness of the disease through *in vitro* differentiation and proliferation, and transplanting the cell into the diseased position of the pancreas. Alternatively, the multipotent stem cell of the present invention may be directly transplanted into the diseased position of the pancreas.

Among the multipotent stem cells of the present invention, as the agent for treating arteriosclerosis, those which contain cells capable of differentiating into vascular cells at high purity are mentioned. The vascular cells include vascular endothelial cells, vascular smooth muscle and the like.

The multipotent stem cell of the present invention can be used for the treatment of arteriosclerosis by obtaining a cell capable of differentiating into the blood vessel of interest depending on the region of arteriosclerosis or the size thereof through *in vitro* differentiation and proliferation, and transplanting the cell into the region of arteriosclerosis. Alternatively, the multipotent stem cell of the present invention may be directly transplanted into the region of arteriosclerosis.

Among the multipotent stem cells of the present invention, those which contain cells capable of differentiating into blood cells at high purity can be mentioned as the agent for treating leukemia. Preferably, the blood cells include hematopoietic stem cells, leukocytes, erythrocytes, platelets and the like.

The multipotent stem cell of the present invention can be used for the treatment of leukemia by obtaining a cell capable of differentiating into the blood cell of interest depending on the necessary amount through *in vitro* differentiation and proliferation, and transplanting the cell into bone marrow. Alternatively, the multipotent stem cell of the present invention may be directly transplanted into bone marrow.

### 6. Preparation of an antibody which recognizes the multipotent stem cell of the present invention

A method for producing an antibody which specifically recognizes the multipotent stem cell of the present invention is described below.

As the antigen, 3 to 5×10⁵ cells/animal of the multipotent stem cell of the present invention or 1 to 10 mg/animal of a cell membrane fraction prepared from the cell is administered together with an appropriate adjuvant (e.g., complete Freund's adjuvant or aluminum hydroxide gel, pertussis vaccine), subcutaneously, intravenously or intraperitoneally to a non-human mammal such as rabbit, goat, or 3-20 week-old rat, mouse or hamster.

After the first administration, the antigen is further administered 3 to 10 times at intervals of 1 to 2 weeks. Three to seven days after each administration, a blood sample is taken from the venous plexus of the fundus of the eye, and the serum derived from the sample blood is tested as to whether it is reactive with the antigen used in the immunization, for example, by enzyme immunoassay [*Enzyme-Linked Immunosorbent Assay (ELISA),* published by Igaku Shoin (1976); *Antibodies-A Laboratory Manual,* Cold Spring Harbor Laboratory (1988)]. A non-human mammal whose serum shows a sufficient antibody titer against the antigen used for immunization is submitted for use as the supply source of serum or antibody-producing cell.

A polyclonal antibody can be prepared by separating and purifying the serum.

A monoclonal antibody can be prepared by preparing a hybridoma through the fusion of the antibody-producing cell with a myeloma cell derived from a non-human mammal and culturing the hybridoma. Alternatively, a monoclonal antibody can be prepared by administering it to an animal to cause ascites tumor of the animal, and then separating and purifying the culture medium or ascites.

The antibody-producing cell preferably used includes splenocyte, an antibody-producing cell in lymph node or peripheral blood, and particularly preferred is splenocyte.

The myeloma cell preferably used includes cell lines such as 8-azaguanine-resistant mouse (BALB/c-derived) myeloma cell lines P3-X63Ag8-U1 (P3-U1) [*Current Topics in Microbiology and Immunology,* 18, 1 (1978)], P3-NS1/1-Ag41 (NS-1) [*European J. Immunology,* 6, 511(1976)], SP2/0-Ag14 (SP-2) [*Nature,* 276, 269 (1978)], P3-X63-Ag8653 (653) [*J. Immunology,* 123 , 1548 (1979)], and P3-X63-Ag8 (X63) [*Nature,* 256, 495 (1975)].

The hybridoma cell can be prepared by the following method.

An antibody-producing cell and a myeloma cell are mixed, suspended in a HAT medium (a medium prepared by supplementing the normal medium with hypoxanthine, thymidine and aminopterin), followed by culturing for 7 to 14 days. After the culturing, a portion of each culture supernatant is taken out, and a sample which reacts with the antigen but does not react with protein containing no antigen is selected by enzyme immunoassay. Subsequently, cloning is carried out by limiting dilution method, and a cell stably showing a high antibody titer by the enzyme immunoassay is selected as a monoclonal antibody-producing hybridoma cell.

The method for separating and purifying the polyclonal antibody or monoclonal antibody includes a method in which a sample is treated by centrifugation, ammonium sulfate precipitation or caprylic acid precipitation, or chromatography using a DEAE-Sepharose column, an anion exchange column, a protein A or G-column, a gel filtration column or the like, alone or in combination thereof.

Whether or not a sample cell expresses a surface antigen specific for the multipotent stem cell of the present invention can be examined by using the obtained antibody capable of specifically recognizing the multipotent stem cell of the present invention.

### 7. Preparation of surface antigen expressing on the multipotent stem cell of the present invention and a gene encoding the surface antigen

The surface antigen gene specifically expressing on the multipotent stem cell of the present invention can be obtained in the following manner by a subtraction method [*Proc. Natl. Acad Sci. USA,* 85, 5738-5742 (1988)] or by representational difference analysis [*Nucleic Acids Research,* 22*,* 5640-5648 (1994)]. The subtraction method is a method for obtaining a gene which takes different expression modes between two samples of different origins.

First, a cDNA library prepared from a multipotent stem cell derived from the interstitial tissues of skeletal muscle is subjected to subtraction using mRNA prepared from a control cell other than the multipotent stem cell of the present invention. After preparing a differentiated cDNA library by concentrating a gene specifically expressing on the multipotent stem cell of the present invention, the nucleotide sequence of the insertion cDNA sequences of the differentiated cDNA library is analyzed at random from the 5' terminal side to select those having a secretion signal sequence alone (random sequence analysis). By determining the full length nucleotide sequence of the obtained cDNA encoding the surface antigen, it is possible to examine whether the protein encoded by the cDNA is a secretory protein or a membrane protein.

In the above method, a signal sequence trap method [*Science,* 261, 600-603 (1993); *Nature Biotechnology,* 17, 487-490 (1999)] can also be used instead of the random sequence analysis. The signal sequence trap method is a method for selectively screening a gene having a secretion signal sequence.

In order to obtain a specific surface antigen efficiently, it is desirable to use a vector capable of carrying out subtraction in preparing a signal sequence trap library derived from the multipotent stem cell of the present invention.

A DNA fragment containing a secretion signal sequence is obtained by carrying out subtraction on the signal sequence trap library prepared from the multipotent stem cell of the present invention, using a mRNA obtained from a control cell. The obtained DNA fragment containing a secretion signal sequence can be used as a probe for cloning the full length cDNA. A full length cDNA encoding the surface antigen can be obtained by using the secretion signal sequence-containing DNA fragment as a probe.

By analyzing the full length nucleotide sequence of the full length cDNA encoding the surface antigen, it is possible to examine whether the protein encoded by the cDNA is a secretory protein or a membrane protein.

Even in the case where the random sequence analysis or signal sequence trap method is used, when the obtained clone encodes a membrane protein, a specific antibody can be obtained by the above method by preparing a synthetic peptide based on an amino acid sequence deduced from the nucleotide sequence and using the synthetic peptide as an antigen.

In addition, in the case of a membrane protein, it may be a receptor. In the case of a receptor, there is a possibility that it is concerned in the control of specific proliferation of a multipotent stem cell or its differentiation into various cells, so that it can be used for the screening of ligand of the receptor. In the case of a secretory protein, it can be used for proliferating or differentiating the multipotent stem cell.

### 8. Method for screening a factor for proliferation of multipotent stem cell and a factor for inducing its differentiation into various cells

The method for screening a factor for proliferation of the multipotent stem cell of the present invention and a factor for inducing differentiation into skeletal muscle cells, smooth muscle cells, cardiomyocytes, blood cells, vascular endothelial cells, adipocytes, osteoblasts, nervous cells, hepatocytes, pancreatic cells and the like can be carried out by adding various substances to be tested in culturing the multipotent stem cell of the present invention in a serum-free medium, and examining whether the cell can grow or whether it is differentiated and induced into cells such as skeletal muscle cells, smooth muscle cells, cardiomyocytes, blood cells, vascular endothelial cell, adipocytes, osteoblasts, nervous cells, hepatocytes and pancreatic cells.

The substances to be tested may be any substances including secretory proteins such as various cytokine and growth factor; membrane-binding proteins such as cell adhesion molecule; tissue extracts; synthetic peptides; synthetic compounds; microbial culture broths; and the like.

The ability of the substances to be tested to proliferate the cell can be measured based on colony forming ability of the cell or incorporation ofBrdU.

The colony forming ability can be examined by inoculating the multipotent stem cell of the present invention at a low density.

Incorporation of BrdU can be examined by immunostaining using an antibody capable of specifically recognizing BrdU.

The ability of the substances to be tested to induce differentiation of the cell into cells such as skeletal muscle cells, smooth muscle cells, cardiomyocytes, blood cells, vascular endothelial cells, adipocytes, osteoblasts, nervous cells, hepatocytes and pancreatic cells can be measured by observing the expression of a marker specifically for each cell or by observing the expression of a reporter gene introduced into cells as an index.

The method using a marker expressing specifically for each cell includes a conventionally known method using an antibody which recognizes a marker of the differentiated cells or a probe such as DNA/RNA *(Immunostaining-In situ Hybridization,* Supplement of Experimental Medicine, edited by Sumiharu Noji, published by Yodosha).

The markers expressing specifically for each cell are exemplified below. The marker which recognizes neuron includes microtuble associate protein 2ab, neurofilament and the like. The marker which recognizes glia includes glial fibrillary acidic protein. The marker which recognizes cardiac muscle includes Mkx2.5, GATA4, cardiac Troponin I and the like. The marker which recognizes muscle includes MyoD and the like. The marker which recognizes adipocyte includes peroxisome proliferation-activated receptor γ2, lipoprotein lipase, fatty acid-binding protein and the like. The marker which recognizes vascular endothelium includes kdr/flk1, low density lipoprotein receptor and the like. The marker which recognizes blood cell includes CD45. The marker which recognizes bone includes alkaline phosphatase. The marker which recognizes cartilage includes type II collagen. The marker which recognizes pancreatic β cell includes insulin. The marker which recognizes hepatocyte includes albumin.

As the method using a reporter gene, mentioned is a method which comprises introducing, into the multipotent stem cell of the present invention, a vector DNA in which a reporter gene is bound to the promoter of a gene specifically expressing in skeletal muscle cells, smooth muscle cells, cardiomyocytes, blood cells, vascular endothelial cells, adipocytes, osteoblasts, nervous cells, hepatocytes, pancreatic cells or the like, and then observing expression of the reporter gene using the cell.

The reporter gene includes GFP (green fluorescent protein), luciferase, β-galactosidase and the like.

### 9. Method for immortalizing the multipotent stem cell of the present invention

When a medicament containing the multipotent stem cell of the present invention is administered to a patient, it is preferable to immortalize the multipotent stem cell of the present invention without causing malignant alteration.

The method for immortalizing the cell without causing malignant alteration includes a method in which telomerase is expressed in the multipotent stem cell of the present invention.

The method for expressing telomerase in the multipotent stem cell of the present invention includes a method in which TERT gene as a catalytic subunit of telomerase, specifically the DNA represented by SEQ ID NO:19, is introduced into a retrovirus vector, and the vector is introduced into the multipotent stem cell.

In addition, telomerase can also be expressed in the multipotent stem cell of the present invention by adding a factor capable of inducing expression of TERT gene existing in the multipotent stem cell of the present invention to a medium used for the culturing of the multipotent stem cell, or by inserting a DNA encoding the factor capable of inducing expression of TERT gene into a vector and introducing the resulting vector into the multipotent stem cell of the present invention.

The factor capable of inducing expression of TERT gene can be screened by adding a compound to be tested directly to the medium to be used for the culturing of the multipotent stem cell and observing expression of telomerase therein as an index. In addition, it can also be screened by inserting the TERT gene and a reporter gene such as GFP (green fluorescent protein), luciferase or β-galactosidase into a vector DNA and introducing the vector into the multipotent stem cell.

The present invention is described below in detail based on examples.

### Best Mode for Carrying Out the Invention

### Example 1

### Analysis of stem cells in rat skeletal muscle:

In order to study a cell which forms new muscle fiber in muscle, plantar muscle of a male Wister rat of 3 weeks of age was analyzed by a histochemical method. After the birth, each rat was raised at 23 ± 1°C at a cycle of 12 hours under light and 12 hours under dark. The rat was sacrificed by injecting pentobarbital chloride to a final concentration of 60 mg/kg, and muscle was excised from the plantar region. The excised muscle was frozen in isopentane cooled with liquid nitrogen and stored at -80°C until its use in the following tests. The muscle tissue sections to be used in the immunohistostaining was prepared by equilibrating the frozen muscle sample at -20°C and then slicing the sample into 6 fragments so that the total muscle tissue can be analyzed. Each section was prepared to a thickness of 7 µM, and 10 to 18 sections were prepared from one tissue fragment.

For the immunostaining, an anti-myogenin antibody as an antibody for skeletal muscle markers (F5D, manufactured by Dako), an anti-MyoD antibody (anti-MyoD1, 5.8A, manufactured by Dako), an anti-myosin heavy chain antibody (manufactured by Novocastra), an anti-laminin antibody as a muscle fiber staining antibody (Chemicon, manufactured by International), an anti-m-cadherin antibody as an antibody for cell adhesion molecules (N-19, manufactured by Santa Cruz) and an anti-CD34 antibody as an antibody for a hematopoietic cell surface antigen (C-18, manufactured by Santa Cruz) were respectively used. As its differentiation progresses, skeletal muscle is expressed on the cell surface as myogenin, MyoD, myosin heavy chain and laminin in that order.

In addition, the immuno response was visualized using a biotin-avidin peroxide reaction.

Myoblast in the plantar muscle of a male Wister rat of 3 weeks of age after birth was analyzed by immunostaining of tissue fragments using various antibodies. As a result, about 70% of the MyoD-positive cells observed in the tissue fragment exist in inside of the basal lamina, that is, they exist in inside of muscle fibers. The remaining 30% of the MyoD-positive cells have small cytoplasm and exist in the interstitial tissues between muscle fibers and muscle fibers. Similar existence of cells was also observed in myogenin-positive cells, but its strength was smaller than that of MyoD-positive cells. The anti-myogenin antibody stained small cells of cytoplasm inside of the muscular fibers and in the interstitium. In a part of the cells, both of myogenin and MyoD were expressed.

In the analyzed sections, 15 to 20 myosin heavy chain-positive small muscle fibers which are observed at the stage of development were detected per one dorsal section. Nuclei of these myosin heavy chain-positive cells were myogenin-positive, and the membrane of myosin heavy chain-positive cell was laminin-positive.

Based on the above results, it is considered that these myosin heavy chain-positive small muscle fibers which are observed at the stage of development are cells which newly form muscle.

Such muscle-forming cells were also observed by an electron microscope analysis.

Laminin-positive cells having small muscle fibers were observed in the periphery of blood vessels in the interstitial tissues, and the location where a new muscle was developed coincided with the location where myogenin-positive and MyoD-positive cells were observed by the above immunostaining. Satellite cells were also observed in muscle fibers.

The above observation results suggest that a stem cell which produces a muscle fiber exists in the interstitial tissues of growing muscle and that satellite cells exist in muscle fibers and taking a role of thickening muscle fibers.

Myoblast in the plantar muscle of a 3 week-old male rat after birth was analyzed by double staining of muscle tissue fragments using an anti-m-cadherin antibody and an anti-laminin antibody. As a result, m-cadherin exists in the inside of muscle fibers where satellite cells exists, but m-cadherin was not observed in the interstitial tissues. Some of the cell surfaces of m-cadherin-positive cells were MyoD-positive, but other cell surfaces were MyoD-negative. The CD34-positive cells were always observed in the interstitium outside of muscle fibers. The existing number of CD34-positive cells was 30 to 40 per one section, and this number was almost the same as the number of MyoD-positive cells. The CD34-positive cells did not co-express MyoD.

In contrast to the fact that the known satellite cells are m-cadherin-positive and CD34-negative, the above results showed that the interstitial stem cell which was found for the first time by this analysis is m-cadherin-negative and CD34-positive.

In addition, since the cell does not express MyoD unlike the case of satellite cells, it was shown that the cell is a multipotent stem cell to which differentiation into muscle is not limited.

### Example 2

### Separation of stem cell from mouse skeletal muscle interstitium:

Interstitial cells of skeletal muscle were excised from a hind leg femoral region of a 3 to 4 week-old C57BL/6 mouse. An obtained muscle piece was minced by finely cutting with scissors and then incubated at 37°C for 2 hours in Dulbecco's modified Eagle's medium (DMEM) containing 0.06% collagenase type IA (manufactured by Sigma) and 10% fetal calf serum. The cells extracted by the treatment were recovered by firstly filtering with a 40 µm nylon mesh, further filtering with a 20 µm nylon mesh, followed by centrifugation at 1,100 rpm for 5 minutes. The cells obtained in this manner were suspended in Dulbecco's modified Eagle's medium (DMEM) containing 20% fetal calf serum to obtain a suspension of skeletal muscle interstitial cells.

In order to obtain a skeletal muscle stem cell from the suspension of skeletal muscle interstitial cells, separation was carried out by using antibodies and a flow cytometry (FACS sorter). In order to remove blood cells contained in the suspension of skeletal muscle interstitial cells, a CD34-positive and CD45-negative fraction (hereinafter referred to as "skeletal muscle interstitial CD34⁺/45⁻ cell") and a CD34-negative and CD45-negative fraction (hereinafter referred to as "skeletal muscle interstitial CD34⁻/45⁻ cell") were separated by using an anti-CD34 antibody (RAM34, manufactured by Pharmingen) and an anti-CD45 antibody (30-F11, manufactured by Pharmingen).

The skeletal muscle interstitial CD34⁺/45⁻ cell was further analyzed by using an antibody which recognizes CD14 which is a hematopoietic cell surface antigen (rmC5-3, manufactured by Pharmingen), an antibody which recognizes CD31 which is a vascular endothelial cell surface antigen (MEC13.3, manufactured by Pharmingen), an antibody which recognizes CD144 which is a vascular endothelial cell surface antigen (11D4.1, manufactured by Pharmingen), an antibody which recognizes FLK-1 which is a vascular endothelial cell surface antigen (Ly-73, manufactured by Pharmingen), an antibody which recognizes CD49d which is an integrin surface antigen (R1-2, manufactured by Pharmingen), and an antibody which recognizes c-kit (2B8, manufactured by Pharmingen) which is a hematopoietic cell surface antigen and an antibody which recognizes Sca-1 (Ly6A/E, manufactured by Pharmingen) which is a hematopoietic cell surface antigen.

As a result, 93.7 +/- 1.3% of the skeletal muscle interstitial CD34⁺/45⁻ cell was Sca-1-positive, and all of the other was negative. The result showed that skeletal muscle interstitial CD34⁺/45⁻ cell does not contain hematopoietic precursor cells or vascular precursor cells.

In addition, since it has been reported that a side-population cell (SP cell) separated from skeletal muscle is CD34-negative, Sca-1-positive, c-kit-negative and CD45-negative [*Nature,* 401, 390-394 (1999)], it was shown that the skeletal muscle interstitial CD34⁺/45⁻ cell is a novel cell which is different from the SP cell.

Next, mRNA was separated from the obtained skeletal muscle interstitial CD34⁺/45⁻ cell using an RNA isolation kit (Isogen, manufactured by Wako) to synthesize a cDNA using RNA PCR kit ver. 2.1 (manufactured by Takara). Using the synthesized cDNA, expression of MyoD, myf-5, myf-6, Myogenin, m-cadherin, c-met, Pax-7, Pax-3 and β-actin was analyzed by RT-PCR. The following synthetic DNA primers were used for the RT-PCR analysis; MyoD (SEQ ID NOs: 1 and 2), myf-5 (SEQ ID NOs:3 and 4), myf-6 (SEQ ID NOs:5 and 6), Myogenin (SEQ ID NOs:7 and 8), m-cadherin (SEQ ID NOs:9 and 10), c-met (SEQ ID NOs:1 1 and 12), Pax-7 (SEQ ID NOs*:*13 and 14), Pax-3 (SEQ ID NOs:15 and 16) and β-actin (SEQ ID NOs:17 and 18).

As a result of the RT-PCR analysis, the skeletal muscle interstitial CD34⁺/45⁻ cell was positive only for c-met, excluding the internal control β-actin, and was negative for all of the other markers.

The result shows that the skeletal muscle interstitial CD34⁺/45⁻ cell is a novel stem cell which is different from the Pax7-positive satellite cell.

### Example 3

### Culturing of skeletal muscle interstitial CD34⁺/45⁻ cell:

In order to analyze differentiation potency of the skeletal muscle interstitial CD34⁺/45⁻ cell obtained in Example 2, the cell was cultured by the following method. The skeletal muscle interstitial CD34⁺/45⁻ cell was cultured by using a complete methyl cellulose medium MethocultGFH 44s4V (manufactured by StemCell Tech) at 1×10⁴ cells/ml. Culturing was carried out at 37°C using an incubator in an atmosphere of 5% CO₂ and 95% O₂. After culturing for 3 days, the skeletal muscle interstitial CD34⁺/45⁻ cell formed a colony comprising spherical cells having a uniform size. The number of cells inside the colony increased with the elapse of time, and a part thereof was released from the culture dish to form a sphere. Also, another part of the cells started to differentiate into small muscle-like cells during a period of the 7th to 10th days and started autonomous movement. Also, it was verified that this cell is a skeletal muscle cell because the cell was stained with an anti-MyoD antibody (5.8A, manufactured by Dako). Also, a vascular endothelial cell-like cell was detected other than the skeletal muscle cell, and this cell was identified as a vascular endothelial cell because it incorporated DiI-Ac-LDL (manufactured by Biochemical Technologies). In addition, an adipocyte which has a drop of oil inside the cell and is stained with Oil red was also detected. The above results shows that the skeletal muscle interstitial CD34⁺/45⁻ cell is a multipotent stem cell capable of differentiating into at least a skeletal muscle, a vascular endothelial cell and an adipocyte.

### Example 4

### Transplantation of skeletal muscle interstitial CD34⁺/45⁻ cell:

In order to elucidate functions of the skeletal muscle interstitial CD34⁺/45⁻ cell in the living body, the skeletal muscle interstitial CD34⁺/45⁻ cell was firstly separated from a GFP transgenic mouse in the same manner as in Example 2. Next, the skeletal muscle interstitial CD34⁺/45⁻ cell derived from the GFP transgenic mouse was transplanted into a front region of the neck bone muscle of an NOD-scid mouse. Six weeks thereafter, the mouse was dissected for analysis, and expression of the GFP protein was observed in muscle fibers and vascular endothelial cells. This result shows that the skeletal muscle interstitial CD34⁺/45⁻ cell can differentiate into skeletal muscle and vascular endothelial cells.

### Example 5

### Separation of skeletal muscle interstitial CD34⁺/45⁻ cell from human skeletal muscle cells:

A total muscle collected from a patient based on the informed consent was made into a mince with scissors or a surgical knife, and then suspended in DMEM (high glucose, manufactured by GIBCO BRL) containing 0.06% collagenase IA (manufactured by Sigma) and 10% FBS, and incubated at 37°C for 2 hours. Cells separated from the minced muscle were recovered by centrifugation and suspended in DMEM (high glucose) containing 10% FBS. The resulting suspension was passed through a microfilter of 40 µm in pore size and then further passed through a microfilter of 20 µm in pore size to obtain a suspension of human skeletal muscle interstitial cells. The obtained cell suspension was subjected to separation of cells in the same manner as in Example 2 by using anti-CD34 antibody and ant-CD45 antibody. As a result, it was observed that 7.54% of the total skeletal muscle interstitial cells was CD34-positive and CD45-negative. This result indicates that the information obtained using mice can also be applied to human.

### Example 6

### Culturing of a stem cell in mouse skeletal muscle interstitium-derived CD34⁻/45⁻ cells:

About 10,000 CD34⁻/45⁻ cells obtained by the method described in Example 2 were cultured in a 35 mm culture dish containing CollagenCult Medium without Cytokines (manufactured by StemCell Technologies Inc.) supplemented with bFGF (10 ng/ml) and EGF (20 ng/ml). As a result, it was observed that about a little over 1% of the cells form colonies of single cells. Also, when the colony-formed cells are continuously cultured, they are stained with an anti-CD34 antibody (RAM34, manufactured by Pharmingen), which shows that CD34⁺/45⁻ cells appear.

When the culturing was further continued, differentiation into skeletal muscle, adipocyte and vascular endothelial cell was observed. Differentiation into skeletal muscle was confirmed by staining with an anti-MyoD antibody (5.8A, manufactured by Dako), differentiation into vascular endothelial cell was confirmed by incorporation of a labeled low density lipoprotein (Dil-Ac-LDL, manufactured by Biomedical Technologies) into the cell, and differentiation into adipocyte by staining with oil-red0 (manufactured by Sigma Aldrich).

The above result shows that the CD34⁺/45⁻ cells are induced from a stem cell contained in CD34⁻/45⁻ cells. It also shows that the cell which has the colony forming ability and is present in the CD34⁻/45⁻ cells is a multipotent stem cell.

In addition, when cells present in the bone marrow interstitium were analyzed in accordance with the method shown in Example 1, CD34-negative, m-cadherin-negative and MyoD-negative cells were observed in addition to the CD34-positive, m-cadherin-negative and MyoD-negative cells. Based on the above, it is considered that the multipotent stem cell having the ability to form colonies and present in the CD34⁻/45⁻ cells has property of being CD34-negative, m-cadherin-negative and MyoD-negative.

### Example 7

### Transplantation of a mouse skeletal muscle interstitium-derived CD34⁺/45⁻ cell under the renal capsule:

About 10,000 CD34⁺/45⁻ cells obtained by the method described in Example 2 were immediately transplanted under the renal capsule of a mouse of the same line, C57BL/6 mouse. Six weeks after the transplantation, the kidney was excised to prepare tissue sections, and conditions of implantation and differentiation of the transplanted cells were observed.

As a result of the observation of the tissue sections with an optical microscope, muscle fibers and a blood vessel, as well as nerve axons having a myelin structure, were found under the renal capsule as morphology of the transplanted cells, which indicates that the CD34⁺/45⁻ cell was differentiated into not only muscle fibers and a blood vessel but also into a nerve, and has multipotency.

The observation of the tissue sections with an optical microscope also confirms the presence of satellite cells in the mature type muscle fibers, which indicates that the CD34⁺/45⁻ cell can also be differentiated into satellite cells.

### Industrial Applicability

Skeletal muscle interstitium-derived multipotent stem cells useful for the regeneration of tissues and cells, and the like are provided.

Free Text of Sequence Listing:
SEQ ID NO:1 - Explanation of synthetic sequence: Synthetic DNA
SEQ ID NO:2 - Explanation of synthetic sequence: Synthetic DNA
SEQ ID NO:3 - Explanation of synthetic sequence: Synthetic DNA
SEQ ID NO:4 - Explanation of synthetic sequence: Synthetic DNA
SEQ ID NO:5 - Explanation of synthetic sequence: Synthetic DNA
SEQ ID NO:6 - Explanation of synthetic sequence: Synthetic DNA
SEQ ID NO:7 - Explanation of synthetic sequence: Synthetic DNA
SEQ ID NO:8 - Explanation of synthetic sequence: Synthetic DNA
SEQ ID NO:9 - Explanation of synthetic sequence: Synthetic DNA
SEQ ID NO:10 - Explanation of synthetic sequence: Synthetic DNA
SEQ ID NO:11 - Explanation of synthetic sequence: Synthetic DNA
SEQ ID NO:12 - Explanation of synthetic sequence: Synthetic DNA
SEQ ID NO:13 - Explanation of synthetic sequence: Synthetic DNA
SEQ ID NO:14 - Explanation of synthetic sequence: Synthetic DNA
SEQ ID NO:15 - Explanation of synthetic sequence: Synthetic DNA
SEQ ID NO:16 - Explanation of synthetic sequence: Synthetic DNA
SEQ ID NO:17 - Explanation of synthetic sequence: Synthetic DNA
SEQ ID NO:18 - Explanation of synthetic sequence: Synthetic DNA

## Claims

1. A multipotent stem cell derived from the interstitial tissues of skeletal muscle.

2. The multipotent stem cell according to claim 1, which is c-met-positive.

3. The multipotent stem cell according to claim 1 or 2, which is CD34-positive.

4. The multipotent stem cell according to any one of claims 1 to 3, which is Scal-positive.

5. The multipotent stem cell according to any one of claims 1 to 4, which is m-cadherin-negative.

6. The multipotent stem cell according to any one of claims 1 to 5, which is CD45-negative.

7. The multipotent stem cell according to any one of claims 1 to 6, which is CD14-negative, CD31-negative, CD49d-negative, CD117-negative, FLK-1-negative and CD144-negative.

8. The multipotent stem cell according to claim 1, which is CD34-negative, CD45-negative, m-cadherin-negative and MyoD-negative.

9. The cell according to any one of claims 1 to 8, which is a multipotent stem cell capable of differentiating into at least skeletal muscle cells.

10. The cell according to any one of claims 1 to 9, which is a multipotent stem cell capable of differentiating into at least a skeletal muscle cell, a smooth muscle cell, a cardiomyocyte, a vascular endothelial cell and an adipocyte.

11. The cell according to any one of claims 1 to 10, which is a multipotent stem cell capable of differentiating into at least a skeletal muscle cell, a smooth muscle cell, a cardiomyocyte, a blood cell, a vascular endothelial cell, an adipocyte, a cartilage cell, an osteoblast, a nervous cell, a hepatocyte, a pancreatic cell, a nephrocyte, a prostatic cell, a mammary gland cell, a small intestine epidermal cell and a corneal cell.

12. The cell according to any one of claims 1 to 11, wherein the skeletal muscle is derived from a mammal.

13. The cell according to claim 12, wherein the mammal is selected from human and rat.

14. A medicament which comprises the cell according to any one of claims 1 to 13.

15. The medicament according to claim 14, wherein the medicament is an agent for regenerating a tissue or a cell.

16. The medicament according to claim 14, wherein the medicament is an agent for treating organ insufficiencies.

17. The medicament according to claim 15, wherein the tissue or cell is a tissue or cell of muscle, heart, blood, blood vessel, bone, joint, pancreas, liver and nerve system.

18. An antibody which specifically recognizes the cell according to any one of claims 1 to 13.

19. A method for purifying the cell according to any one of claims 1 to 13 from human skeletal muscle, which comprises using the antibody according to claim 18.

20. A method for screening a substance which proliferates the cell according to any one of claims 1 to 13, which comprises using the cell.

21. A method for screening a substance which induces differentiation of the cell according to any one of claims 1 to 13 into various cells by the cell according to any one of claims 1 to 13.

22. The screening method according to claim 21, wherein the various cells are selected from a skeletal muscle cell, a smooth muscle cell, a cardiomyocyte, a blood cell, a vascular endothelial cell, an adipocyte, an osteoblast, a cartilage cell, a nervous cell, a hepatocyte, a pancreatic cell, a nephrocyte, a prostatic cell, a mammary gland cell, a small intestine epidermal cell, a skin cell and a corneal cell.

23. A method for immortalizing the cell according to any one of claims 1 to 13.

24. A method for separating the cell according to any one of claims 1 to 13, wherein a sphere is formed.
